(19) Europäisches Patentamt
European Patent Office
Office européen des brevets

(11) Veröffentlichungsnummer: **0 214 102 B1**

(12) **EUROPÄISCHE PATENTSCHRIFT**

(45) Veröffentlichungstag der Patentschrift: **23.05.90**

(21) Anmeldenummer: **86810388.8**

(22) Anmeldetag: **28.08.86**

(51) Int. Cl.$^5$: **C 07 D 249/20,** C 08 K 5/34, C 07 C 245/24

# (54) 5-Aralkylsubstituierte 2H-Benztriazole und stabilisierte Zusammensetzungen.

(30) Priorität: **03.09.85 US 772221**

(43) Veröffentlichungstag der Anmeldung:
**11.03.87 Patentblatt 87/11**

(45) Bekanntmachung des Hinweises auf die Patenterteilung:
**23.05.90 Patentblatt 90/21**

(84) Benannte Vertragsstaaten:
**BE DE FR GB IT NL**

(56) Entgegenhaltungen:
**EP-A-0 130 938**
**US-A-4 278 589**
**US-A-4 477 614**
**US-A-4 487 805**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Seltzer, Raymond**
**11 Angus Lane**
**New City New York 10956 (US)**
Erfinder: **Winter, Roland A.E.**
**23 Banksville Road**
**Armonk New York 10504 (US)**

(56) Entgegenhaltungen:
**CHEMICAL ABSTRACTS, Band 82, Nr. 7, 17. Februar 1975, Columbus, Ohio, USA BELUSA, J.; MAZANKOVA, J.; KOVACIK, V.; RABUSIC, E.; POTACEK, M. "2-(2-Hydroxyphenyl)-benzotriazoles. II. Eletrophilic substitution reactions on the molecule of 2-(2-hydroxy-5-methylphenyl)benzotriazole and ultraviolet spectra of the products" Seite 428, Spalte 2, Zusammenfassung-Nr. 43 277f**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 214 102 B1

**Beschreibung**

Vorliegende Erfindung betrifft ausgewählte 2-Aryl-2H-benztriazole, die zum Schutz lichtempfindlicher organischer Materialien gegen Abbau verwendbar sind, sowie stabilisierte, solche Benztriazole enthaltende Zusammensetzungen.

Die UV-Absorber aus der Hydroxyphenyl-2H-benztriazolklasse sind seit langem als wirksame Lichtschutzmittel für organische Materialien bekannt und haben sich im Handel weitgehend durchgesetzt.

Die bisher bakannten 2-Aryl-2H-benztriazole aus dieser Gruppe zeigten jedoch unter gewissen Umständen eine begrenzte Verträglichkeit in bestimmten Substraten sowie eine zu grosse Neigung zum Ausschwitzen, Sublimieren und/oder Verflüchtigen bei der Verarbeitung der stabilisierten Zusammensetzungen zu Folien, Fasern oder sonstigen Flächengebilden, wenn die Verarbeitung bei erhöhten Temperaturen durchgeführt werden muss. Ferner können solche Benztriazole auch übermässige Verluste durch Verflüchtigung oder Sublimation aus fertig bearbeiteten Gebilden, insbesondere dünnen Filmen oder Überzügen, erleiden, und zwar wenn sie bei der Verwendung erhöhten Temperaturen ausgesetzt sind.

Es wurden schon Versuche unternommen, durch Abänderung der Struktur der Benztriazole die Substratverträglichkeit oder -löslichkeit zu erhöhen und den Verflüchtigungsverlust zu verringern.

In U.S. Patent 3 230 194 wurde Methyl im Hydroxyphenylteil durch eine höhere Alkylgruppe ersetzt.

Noch weitere Abänderungen am 2-Aryl-2H-benztriazolteil wurden im U.S. Patent 4 127 586 gemacht.

Aus der japanischen Offenlegungsschrift 158 588/75 sind weitere Benztrizollichtschutzmittel wie 2-(2-Hydroxy-3-α,α-dimethylbenzyl 5-methylphenyl)-2H-benztriazol bekannt.

Weiter verbesserter Widerstand gegen Verlust aus stabilisierten Zusammensetzungen bei der Hochtemperaturverarbeitung stellt jedoch immer noch ein praktisches Ziel und eine Notwendigkeit in der Technik der Benztriazol-UV-Absorber dar.

Im U.S. Patent 4 226 763 sind Versuche beschrieben, den Widerstand von Benztriazol-Lichtschutzmitteln gegen Verflüchtigungsverlust zu erhöhen. Dieses Patent offenbart 2-(2-Hydroxy-3,5-di-α-cumylphenyl)-2H-benztriazol, das gegenüber die 2-Aryl-2H-benztriazole des Standes der Technik enthaltenden stabilisierten Zusammensetzungen einen überlegenen Widerstand gegen Verlust aus stabilisierten Zusammensetzungen bei der Hochtemperaturverarbeitung oder Endanwendungen aufweist, wo Überzüge oder Filme aus den stabilisierten Zusammensetzungen sogar gewöhnlicher Bewitterung und Belichtung ausgesetzt sind. Um diese überlegene Leistung zu erzielen, muss man eine verhältnismässig niedrige Löslichkeit in einigen Substraten und Verarbeitungslösungsmitteln in Kauf nehmen.

Das in U.S. Patent 4 283 327 beschriebene 2-(2-Hydroxy-3,5-di-tert.octylphenyl)-2H-benztriazol weist erhöhte Löslichkeit in Verarbeitungslösungsmitteln und Substraten, aber keinen hervorragenden Widerstand gegen Verlust durch Verflüchtigung auf.

Die EP—A—130938 beschreibt ein Verfahren zur Herstellung von 2-Hydroxyphenylbenztriazolen, die im Phenolring u.a. mit Aralkylgruppen substituiert sein können.

Aus U.S. Patent 4 278 589 sind Benztriazole mit einer α-Cumylgruppe und einem tert.Octylsubstituenten am 2-Phenylteil bekannt, die ein Eigenschaftsgleichgewicht erzielen sollen, das man mit zwei α-Cumyl- oder zwei tert.-Octylgruppen nicht erhält. Bei diesen Benztriazolen sind die Löslichkeit und der Widerstand gegen Verflüchtigungsverlust zwar gut ausgewogen, doch erreichen dabei nicht beide das hervorragende Niveau, das von einem immer anspruchsvolleren Markt für Lichtschutzmittel mit wirklich aussergewöhnlichen Eigenschaften gefordert wird.

Niederalkyl-, Niederalkoxy- und Halogensubstitution am Benzoring in 2H-Benztriazolen ist zwar schon lange bekannt, beispielsweise aus U.S. Patent 4 129 586, doch ist Substitution am Benzoring mit Benzyl,α-Methylbenzyl,α,α-Dimethylbenzyl oder anderen Phenylalkylgruppen bisher unbekannt. Die japanische Offenlegungsschrift Sho 59/172 655 offenbart generisch eine solche Substitution, beschreibt aber speziell nur eine Aralkylsubstitution am 2-Phenylring von 2-Aryl-2H-benztriazolstabilisatoren.

Herkömmlicherweise wurden zur Erzeugung von Hochglanzanstrichen in der Automobil- und anderen Industrie(n) Lackfarben eingesetzt. Diese bestehen typischerweise aus hochmolekularen, in entsprechenden Lösungsmitteln gelösten Polymeren. Die Lösungsmittel stellen üblicherweise mehr als 70% der Farb dar und verdunsten beim Einbrennen, wobei ein Polymerfilm zurückbleibt.

Energie- und Umweltbetrachtungen haben kürzlich zur Entwicklung sogenannter "Einbrennlacke mit hohen Feststoffgehalt" als alternative Anstrichsystem geführt, welche die staatlich verlangte Verringerung an flüchtigen organischen Verbindungen erreichen. Einbrennlacke hohen Feststoffgehalts bestehen typischerweise aus niedermolekularen Copolymeren aus Methylmethacrylat, Hydroxyethylmethacrylat, Butylacrylat und Styrol. Diese Copolymeren, welche Hydroxylseitengruppen enthalten, werden dann mit Melaminvernetzungsharzen in Verhältnissen von etwa 7:3 vermischt. Die abschliessende Vernetzungreaktion erfolgt, wenn der lackierte Artikel dem Einbrennen unterzogen wird. Im Gegensatz zu Lackfarben enthalten Einbrennlacke hohen Feststoffgehalts gewöhnlich weniger als 50% Lösungsmittel.

Der Hauptteil dieser Lösungsmittel wird während des Polymerisationsverfahrens eingesetzt. Nur eine geringe Lösungsmittelmenge, im allgemeinen weniger als 10% des Gesamtlösungsmittels, wird als "Ausgiebigkeits"-Lösungsmittel zurückgehalten, das dann später der Farbe zugesetzt werden soll. Die Lichtschutzzusätze müssen in diesem Ausgiebigkeits-Lösungsmittel genügend löslich sein, damit sie in dieser Stufe eingebracht werden können. Die Lösungsmittelmenge ist nicht beliebig veränderlich, da die

Viskosität der Farbe eine kristische Grösse zur Vermeidung von Fehlern, wie Verlaufen und Ablaufen, darstellt. Die vorliegenden Stabilisatoren wurden unter anderem entwickelt, um diese Forderungen nach hoher Löslichkeit zur erfüllen. Ferner sind diese Produkte den Materialien des Standes der Technik bezüglich Verträglichkeit mit dem Harz und fehlender Flüchtigkeit gleichwertig und/oder überlegen.

Gegenstand vorliegender Erfindung sind ausgewählte 2-(2-Hydroxyphenyl)-2H-benztriazol-lichtschutzmittel sowie damit stabilisierte organische Materialien.

Die erfindungsgemässen 2-(2-Hydroxyphenyl)-2H-benztriazole entsprechen der Formel I

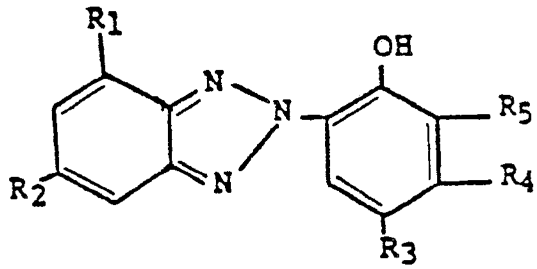

(I)

worin $R_1$ und $R_2$ unabhängig voneinander Wasserstoff, Halogen, Alkyl mit 1 bis 18 Kohlenstoffatomen oder eine Gruppe der Formel II

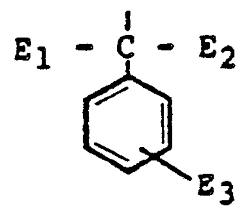

(II)

wobei $E_1$ und $E_2$ unabhängig voneinander für Wasserstoff oder Alkyl mit 1 bis 4 Kohlenstoffatomen und $E_3$ für Wasserstoff, Halogen oder Alkyl mit 1 bis 4 Kohlenstoffatomen stehen, mit der Massgabe, dass mindestens eines von $R_1$ und $R_2$ eine Gruppe der Formel II sein muss, $R_3$ Wasserstoff, Hydroxyl, Alkyl mit 1 bis 12 Kohlenstoffatomen, Alkoxy mit 1 bis 4 Kohlenstoffatomen, gegebenenfalls durch eine, zwei oder drei Alkylgruppen mit 1 bis 8 Kohlenstoffatomen substituiertes Phenyl, Cycloalkyl mit 5 bis 6 Kohlenstoffatomen, Carboalkoxy mit 2 bis 9 Kohlenstoffatomen, Chlor, Carboxyethyl oder eine Gruppe der Formel II, $R_4$ Wasserstoff, Alkyl mit 1 bis 4 Kohlenstoffatomen, Alkoxy mit 1 bis 12 Kohlenstoffatomen, Chlor oder Hydroxyl und $R_5$ Wasserstoff, Hydroxyl, Alkyl mit 1 bis 12 Kohlenstoffatomen, Chlor, Cycloalkyl mit 5 bis 6 Kohlenstoffatomen oder eine Gruppe der Formel II bedeuten, mit der Massgabe, dass $R_3$, $R_4$ und $R_5$ nicht gleichzeitig alle für Wasserstoff stehen dürfen, und dass jeweils nur eines von $R_3$, $R_4$ und $R_5$ Hydroxyl darstellen kann.

Bedeuten $R_1$, $R_2$ oder $E_3$ Halogen, so handelt es sich um Fluor, Brom, Chlor oder Jod, vorzugsweise Chlor.

Bedeuten $R_1$ oder $R_2$ Alkyl mit 1 bis 18 Kohlenstoffatomen, so handelt es sich beispielsweise um Methyl, Ethyl, n-Propyl, Isopropyl, n-Butyl, sek-Butyl, Isobutyl, tert.Butyl, tert.Amyl, 2-Ethylhexyl, n-Octyl, tert.-Octyl, n-Dodecyl, tert.Dodecyl oder n-Octadecyl.

$E_1$, $E_2$ oder $E_3$ als Alkyl mit 1 bis 4 Kohlenstoffatomen sind beispielsweise Methyl, Ethyl, n-Propyl, Isopropyl, n-Butyl oder tert.Butyl.

$R_3$ kann Alkyl mit 1 bis 12 Kohlenstoffatomen wie Methyl, Ethyl, sek-Butyl, tert.Butyl, tert.Amyl, tert.-Octyl oder n-Dodecyl darstellen. Ferner kann $R_3$ Alkoxy mit 1 bis 4 Kohlenstoffatomen wie Methoxy, Ethoxy oder n-Butoxy sein. Auch bedeutet $R_3$ durch eine, zwei oder drei Alkylgruppen substituiertes Phenyl, wobei diese Alkylgruppen 1 bis 8 Kohlenstoffatome aufweisen, wie Methyl, Ethyl, sek.-Butyl, tert.Butyl, tert.Amyl oder tert.Octyl. Ebenfalls kann als $R_3$ Cycloalkyl mit 5 bis 6 Kohlenstoffatomen, wie Cyclopentyl oder Cyclohexyl vorliegen. Auch steht $R_3$ für Carboalkoxy mit 2 bis 9 Kohlenstoffatomen, wie Carbomethoxy, Carboethoxy, Carbo-n-butoxy oder Carbo-n-octoxy. Zudem kann $R_3$ eine Gruppe der Formel II wie Benzyl, α-Methylbenzyl oder α,α-Dimethylbenzyl (=α-Cumyl) bedeuten.

$R_4$ kann Niederalkyl mit 1 bis 4 Kohlenstoffatomen wie Methyl, Ethyl, n-Butyl oder tert.Butyl sein.

$R_4$ kann ferner für Alkoxy mit 1 bis 12 Kohlenstoffatomen wie Methoxy, Ethoxy, n-Butoxy, Octyloxy oder Dodecyloxy stehen.

Als $R_5$ kann Alkyl mit 1 bis 12 Kohlenstoffatomen wie Methyl, Ethyl, sek.Butyl, tert.Butyl, tert.Amyl, tert.Octyl, n-Dodecyl oder tert.Dodecyl vorliegen.

$R_5$ kann ferner Cycloalkyl mit 5 bis 6 Kohlenstoffatomen wie Cyclopentyl oder Cyclohexyl sowie eine Gruppe der Formel II wie Benzyl, α-Methylbenzyl oder α,α-Dimethylbenzyl sein.

Bevorzugt werden solche Verbindungen, worin nur eines von $R_1$ und $R_2$ eine Gruppe der Formel II darstellt, und solche sind besonders bevorzugt, worin $R_1$ Wasserstoff und $R_2$ eine Gruppe der Formel II bedeutet.

Ganz besonders bevorzugt sind die Verbindungen, worin $R_1$ Wasserstoff, $R_2$ eine Gruppe der Formel II, worin mindestens eines von $E_1$ und $E_2$ für Methyl und $E_3$ für Wasserstoff oder Alkyl mit 1 bis 4 Kohlenstoffatomen, insbesondere Wasserstoff oder p-Methyl, stehen, bedeuten. $R_2$ ist ganz besonders α,α-Dimethylbenzyl.

Vorzugsweise bedeutet $R_3$ Alkyl mit 1 bis 8 Kohlenstoffatomen oder eine Gruppe der Formel II.

Vurzugsweise bedeutet $R_4$ Wasserstoff, Hydroxyl, Alkyl mit 1 bis 4 Kohlenstoffatomen oder Alkoxy mit 1 bis 8 Kohlenstoffatomen.

Vorzugsweise bedeutet $R_5$ Wasserstoff, Alkyl mit 1 bis 8 Kohlenstoffatomen oder eine Gruppe der Formel II.

Besonders bevorzugt bedeutet $R_3$ Methyl, tert.Butyl, tert.Amyl, tert.Octyl, sek-Butyl, Benzyl, α-Methylbenzyl oder α,α-Dimethylbenzyl.

Besonders bevorzugt bedeutet $R_4$ Wasserstoff.

Besonders bevorzugt bedeutet $R_5$ Wasserstoff, Methyl, sek.Butyl, tert.Butyl, tert.Amyl, tert.Octyl, Benzyl, α-Methylbenzyl oder α,α-Dimethylbenzyl.

Besonders interessante Verbindungen innerhalb des Umfangs der Formel I sind solche, worin $R_1$ und $R_2$ unabhängig voneinander Wasserstoff, Alkyl mit 1 bis 12 Kohlenstoffatomen oder eine Gruppe der Formel II, wobei $E_1$ und $E_2$ unabhängig voneinander für Wasserstoff, Methyl oder Ethyl und $R_3$ für Wasserstoff, Chlor, Methyl oder Ethyl stehen, $R_3$ Wasserstoff, Alkyl mit 1 bis 12 Kohlenstoffatomen oder eine Gruppe der Formel II, $R_4$ Wasserstoff und $R_5$ Wasserstoff, Alkyl mit 1 bis 12 Kohlenstoffatomen oder eine Gruppe der Formel II bedeuten.

Die erfindungsgemässen Verbindungen werden beispielsweise auf die im U.S. Patent 4 226 763 angegebene Art und Weise hergestellt, bei der das substituierte 2-Nitroanilin (z.B. mit Natriumnitrit) diazotiert und dann vorzugsweise in einem stark alkalischen Medium mit dem entsprechenden Phenol zu dem o-Nitroazobenzolzwischenprodukt gekuppelt wird.

Dieses o-Nitroazobenzolzwischenprodukt wird durch reduktiven Ringschluss unter Verwendung beliebiger herkömmlicher reduzierender Systeme, z.B. Zink und Alkali, Hydrazin, katalytische Hydrierung und dergleichen, in das entsprechende 2-Aryl-2H-Benztriazol überführt. Diese Herstellungsmethode lässt sich durch nachfolgende Reaktionsgleichung veranschaulichen:

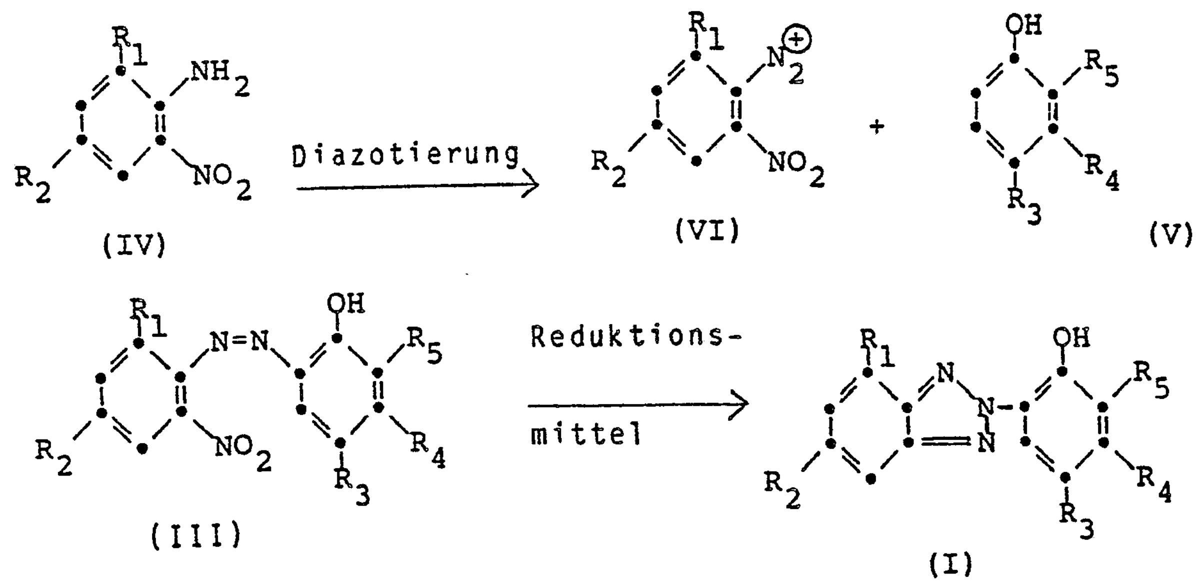

(IV) (VI) (V) (III) (I)

Die substituierten Phenole der Formel V sind bekannt oder lassen sich nach an sich bekannten Methoden herstellen, beispielsweise durch Alkylierung eines Phenols mit einem Olefin in Gegenwart eines sauren Katalysators. Die Herstellung von 2,4-Di-(α,α-dimethylbenzyl)-phenol wie in U.S. Patent 4 226 763 beschrieben ist ein typisches Beispiel.

Die substituierten, zur Herstellung der vorliegenden, am Benzoring mit einer Gruppe der Formel II, beispielsweise mit Benzyl-, α-Methylbenzyl- oder α-Cumyl- (=α,α-Dimethylbenzyl)-Gruppen, substituierten Verbindungen erforderlichen o-Nitroaniline der Formel V sind nach an sich bekannten Methoden erhältlich, zum Beispiel durch Aralkylierung eines o-Nitroanilins unter Verwendung beispielsweise eines Olefins (wie Styrol oder α-Methylstyrol), eines Alkohols (wie Benzylalkohol) oder eines Esters (α-Cumylacetat) in Gegenwart eines sauren Katalysators. Einzelheiten zur Herstellung der Ausgangsstoffe der Formeln IV und V finden sich in den Beispielen.

Viele der verschiedenen Ausgangsstoffe wie die substituierten Phenole, o-Nitroanilin, α-Methylstyrol, 5-Chlor-2-nitroanilin, Styrol, Benzylalkohol und dergleichen sind im Handel erhältlich oder leicht nach bekannten Methoden herstellbar.

Die o-Nitroazobenzolzwischenprodukte der Formel III

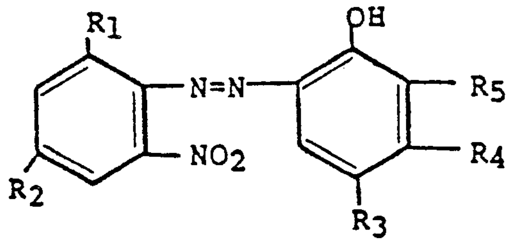
(III)

worin $R_1$, $R_2$, $R_3$, $R_4$ und $R_5$ die oben angegebenen Bedeutungen haben, sind ebenfalls neue Verbindungen und stellen einen Teil dieser Erfindung dar. Die bevorzugten Substituenten R sind dieselben wie oben für die Verbindungen der Formel I erwähnt.

Die erfindungsgemässen Verbindungen der Formel I sind wirksame Lichtschutzmittel in einem weiten Bereich organischer Polymerer. Zu stabilisierbaren Polymeren gehören:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Propfcopolymere von Styrol, wie z.B. Styrol auf Polybutadien, Styrol und Acrylnitril auf Polybutadien, Styrol und Alkylacrylate oder -methacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfonierts Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetate.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymer von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten.

13. Polyphenylenoxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder Aminocarbonsäuren oder den entsprechenden Lactamen ableiten, wie Polyamid 4, Polyamid 6, Polyamid 6/6, Polyamid

6/10, Polyamid 11, Polyamid 12, Poly-2,4,4-trimethylhexamethylenterephthalamid, Poly-m-phenylenisophthalamid, sowie deren Block-Copolymere mit Polyethern, wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-[2,2-(4-hydroxyphenyl)-propan]-terephthalat und Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B von Epoxyacrylaten, Urethan-acrylaten oder Polyester-acrylaten.

24. Alkydharze, Polyesterharze und Acrylatharze, die mit Melaminharzen, Harstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren, wie z.B. PP/EPDM, Polyamid 6/EPDM oder ABS, PVC/EVA, PVC/ABS, PVC/MBS, PC/ABS, PBTP/ABS.

Während die erfindungsgemässen Verbindungen sehr wirksame Stabilisatoren für zahlreiche organische, lichtinduziertem Abbau unterliegende Substrate darstellen, wie auch die 2-Aryl-2H-benztriazollichtabsorber im allgemeinen, sind die vorliegenden Verbindungen mit ihrere überraschenden Widerstandsfähigkeit gegen Verlust durch Verflüchtigung, Ausschwitzen oder Sublimation aus einer stabilisierten Zusammensetzung während der Hochtemperaturverarbeitung besonders wertvoll bei der Stabilisierung polymerer Substrate, die zwangsläufig bei erhöhten Temperaturen verarbeitet werden müssen.

Somit sind die erfindungsgemässen Verbindungen besonders nützlich als Stabilisatoren zum Schutz von Polyestern, z.B. poly-(ethylenterephthalat), Poly-(butylenterephthalat) oder deren Copolymeren, Polycarbonaten, z.B. solchen, die sich von Bisphenol-A und Phosgen ableiten, oder deren Copolymeren, oder Polysulfonen oder Polyamiden wie Polyamid-6, Polyamid-6,6, Polyamid-6,10 und dergleichen sowie Copolyamiden, heisshärtbaren Acrylharzen, thermoplastischen Acrylharzen, Polyolefinen wie Polyethylen, Polypropylen, Copolyolefinen und dergleichen sowie jeglichem Polymersystem, das Verarbeitung und Fertigung bei hohen Temperaturen erfordert.

Besonders zu erwähnen ist auch die Stabilisierung von Aminoplasten mit Hilfe der Verbindungen der Formel I.

Die erfindungsgemässen Verbindungen sind zwar, wie oben erwähnt, ausgezeichnete Lichtschutzmittel, doch werden sie bei der Herstellung erfindungsgemässer stabilisierter Zusammensetzungen häufig mit weiteren Stabilisatoren, gegebenenfalls auch mit weiteren Lichtschutzmitteln kombiniert. Sie können zusammen mit phenolischen Antioxidantien, Pigmenten, Färbemitteln oder Farbstoffen, Lichtschutzmitteln wie sterisch gehinderten Aminen, Metalldesaktivatoren usw. eingesetzt werden.

Die erfindungsgemässen Verbindungen werden im allgemeinen in Mengen von etwa 0,05 bis etwa 10 Gew.-%, insbesondere 0,1 bis etwa 5 Gew.-%, der stabilisierten Zusammensetzung eingesetzt, doch kann dies je nach dem jeweiligen Substrat und der Anwendung schwanken. Ein bevorzugter Bereich liegt zwischen etwa 0,5 und etwa 3%.

Die Stabilisatoren der Formel I lassen sich leicht nach üblichen Methoden in irgendeiner zweckmässigen Stufe vor der Herstellung von Formkörpern daraus in die organischen Polymeren einarbeiten. Beispielsweise kann man den Stabilisator mit dem Polymer in trockener Pulverform oder eine Suspension oder Emulsion des Stabilisators mit einer Lösung, Suspension oder Emulsion des Polymeren vermischen. Die erfindungsgemässen stabilisierten Polymerzusammensetzungen können gegebenenfalls noch verschiedene herkömmliche Zusatzstoffe wie die folgenden, insbesondere phenolische Antioxidantien oder Lichtschutzmittel oder deren Gemische enthalten (beispielsweise in einer Konzentration von etwa 0,1 bis etwa 5 Gew.-%, vorzugsweise von etwa 0,5 bis etwa 3 Gew.-%):

1. Antioxidantien

*1.1 Alkylierte Monophenole*, z.B. 2,6-Di-tert.butyl-4-methylphenol, 2-tert.Butyl-4,6-dimethylphenol, 2,6-Di-tert.butyl-4-ethylphenol, 2,6-Di-tert.butyl-4-n-butylphenol, 2,6-Di-tert.butyl-4-butylphenol, 2,6-Di-cyclo-

pentyl-4-methylphenol, 2-(α-methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol.

*1.2. Alkylierte Hydrochinone*, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

*1.3. Hydroxylierte Thiodiphenylether*, z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis(6-tert.butyl-2-methylphenol).

*1.4. Alkyliden-Bisphenole*, z.B. 2,2'-Methylen-bis-(6-tert.butyl-4-methylphenol), 2,2'-Methylen-bis-(6-tert.butyl-4-ethylphenol), 2,2'-Methylen-bis[4-methyl-6-(-methylcyclohexyl)-phenol], 2,2'-Methylen-bis-(4-methyl-6-cyclohexylphenol), 2,2'-Methylen-bis(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4-Methylen-bis-(2,6-di-tert.butylphenol), 4,4'-Methylen-bis-(6-tert.butyl-2-methylphenol), 1,1-bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglykol-bis-[3,3-bis(3'-tert.butyl-4'-hydroxyphenyl)-butyrat], Di-(3-tert.butyl-4-hydroxy-5-methylphenyl)dicyclopentadien, bis-[2-(3'-tert.butyl-2'-hydroxy-5'-methylbenzyl)-6-tert.butyl-4-methylphenyl]-terephthalat.

*1.5. Benzylverbindungen*, z.B. 1,3,5-Tris-(3,5-di-tert.butyl-4-hydroxybenzyl)-2,4,6-trimethylbenzol, Di-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl) dithiol-terephthalat, 1,3,5-Tris-(3,5-di-tert.-butyl-4-hydroxybenzyl)-isocyanurat, 1,3,5-Tris-(4-tert.butyl-3-hydroxy-2,6-dimethylbenzyl)-isocyanurat, 3,5-Ditert.-butyl-4-hydroxybenzyl-phosphonsäuredioctadecylester, 3,5-Di-tert.butyl-4-hydroxybenzyl-phosphonsäure-monoethylester, Calcium-salz.

*1.6. Acylaminophenole*, z.B. 4-Hydroxy-laurinsäureanilid, 4-Hydroxystearinsäureanilid, 2,4-Bis-octylmercapto-6-(3,5-di-tert.butyl-4-hydroxyanilino)-s-triazin, N-(3,5-di-tert.butyl-4-hydroxyphenyl)-carbaminsäure-octylester.

*1.7. Ester der β-(3,5-Di-tert.butyl-4-hydroxyphenyl)-propionsäure* mit ein- oder mehrwertigen Alkoholen, wie z.B. mit Methanol, Octadecanol, 1,6-Hexandiol, Neopentylglykol, Thiodiethylenglykol, Diethylenglykol, Triethylenglykol, Pentaerythrit, Tris-hydroxyethyl-isocyanurat, Di-hydroxyethyl-oxalsäurediamid.

*1.8 Ester der β-(5-tert.butyl-4-hydroxy-3-methylphenyl)-propionsäure* mit ein- oder mehrwertigen Alkoholen, wie z.B. mit Methanol, Octadecanol, 1,6-Hexandiol, Neopentylglykol, Thiodiethylenglycol, Diethylenglykol, Triethylenglykol, Pentaerythrit, Tris-hydroxyethyl-isocyanurat, Di-hydroxyethyl-oxalsäurediamid.

*1.9. Amide der β-(3,5-Di-tert.butyl-4-hydroxyphenyl)-propionsäure*, wie z.B. N,N'-Di-(3,5-di-tert.butyl-4-hydroxyphenylpropionyl)-hexamethylendiamin, N,N'-Di-(3,5-di-tert.-butyl-4-hydroxyphenylpropionyl)-trimethylendiamin, N,N'-Di-(3,5-di-tert.butyl-4-hydroxyphenylpropionyl)-hydrazin.

2. UV-Absorber und Lichtschutzmittel

*2.1. 2-(2'-Hydroxyphenyl)-benztriazole*, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

*2.2 2-Hydroxybenzophenone*, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-Dimethoxy-Derivat.

*2.3 Ester von gegebenenfalls substituierten Benzoesäuren*, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

*2.4. Acrylate*, wie z.B. α-Cyan-β,β-diphenylacrylsäure-ethylester bzw. -isooctylester, α-Carbomethoxy-zimtsäuremethylester, α-Cyan-β-methyl-p-methoxy-zimtsäuremethylester bzw. -butylester, α-Carbomethoxy-p-methoxy-zimtsäure-methylester, N-(β-Carbomethoxy-β-cyanvinyl)-2-methyl-indolin.

*2.5. Nickelverbindungen*, wie z.B. Nickelkomplexe des 2,2'-Thio-bis-[4-(1,1,3,3,-tetramethylbutyl)-phenols], wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methylphenyl-undecylketonoxim, Nickelkomplexe des 1-Phenyl-4-lauroyl-5-hydroxy-pyrazols, gegebenenfalls mit zusätzlichen Liganden.

*2.6. Sterisch gehinderte Amine*, wie z.B. Bis-(2,2,6,6-tetramethyl-piperidyl)-sebacat, Bis-(1,2,2,6,6-pentamethylpiperidyl)-sebacat, n-Butyl-3,5-di-tert.-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl-bis-(3,3,5,5-tetramethyl-piperazinon).

*2.7. Oxalsäurediamide*, wie z.B. 4,4'-Di-octyloxy-oxanilid, 2,2'-Dioctyloxy-5,5'-di-tert.butyl-oxanilid,

2,2′-Di-dodecyloxy-5,5′-di-tert.butyl-oxanilid, 2-ethoxy-2′-ethyl-oxanilid, N,N′-Bis-(3-dimethylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o- und p-Methoxy- sowie von o- und p-Ethoxy-di-substituierten Oxaniliden.

*3. Metalldesaktivatoren*, wie z.B. N,N′-Diphenyloxalsäurediamid, N-Salicylal-N′-salicyloylhydrazin, N,N′-Bis-salicyloylhydrazin, N,N′-Bis-(3,5-di-tert.butyl-4-hydroxyphenylpropionyl)-hydrazin, 3-Salicyloyl-amino-1,2,4-triazol, Bis-benzyliden-oxalsäuredihydrazid.

*4. Phosphite und Phosphonite*, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Disodecylpentaerythritdiphosphit, Di(2,4-di-tert.butylphenyl)pentaerythritdiphosphit, Tristearyl-sorbit-triphosphit, Tetrakis-(2,4-di-tert.butylphenyl)-4,4′-biphenylen-diphosphonit, 3,9-Bis-(2,4-ditert.butylphenoxy-2,4,8,10-tetraoxa-3,9-diphosphaspiro[5.5]-undecan.

*5. Peroxidzerstörende Verbindungen*, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dode-cylmercapto)-propionat.

*6. Polyamidstabilisatoren*, wie z.B. Kupfersalze in Kombination mit Jodiden und/oder Phosphorverbindungen und Salze des zweiwertigen Mangans.

*7. Basische Co-stabilisatoren*, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, MG-Stearat, Na-Riconoleat, K-Palmitat, Antimonbrenzcatechinat oder Zinkbrenzcatechinat.

*8. Nukleierungsmittel*, wie z.B. 4-tert.Butylbenzoesäure, Adipinsäure, Diphenylessigsäure.

*9. Füllstoffe und Verstärkungsmittel*, wie z.B. Calciumcarbonat, Silikate, Glasfasern, Asbest, Talk, Koalin, Glimmer, Bariumsulfat, Metalloxyde und -hydroxide, Russ, Graphit.

*10. Sonstige Zusätze*, wie z.B. Weichmacher, Gleitmittel, Emulgatoren, Pigmente, optische Aufheller, Flammschutzmittel, Antistatika, Treibmittel.

Die nachfolgenden Beispiele dienen nur der Erläuterung und sind keinesfalls so auszulegen, dass sie die Art oder den Umfang der vorliegenden Erfindung in irgendeiner Weise einschränken.

## Beispiel 1

4-α-Cumyl-2-nitroanilin

In einem mit Kühler, Stickstoffeinlass, Rührer, Thermometer und Tropftrichter ausgerüsteten 12 Liter-Reaktionskolben legt man 187.4 g (1,375 Mol) wasserfreies Zinkchlorid vor, setzt, den Kolben unter Stickstoff und gibt im Verlauf von 10 Minuten 459 ml (5,5 Mol) konzentrierte (12-n) Salzsäure dazu, wobei die Temperatur von 22 auf 40°C steigt. Danach setzt man im Verlauf von 15 Minuten 760 g (5,5 Mol) o-Nitroanilin unter Vermeidung von Klumpenbildung dazu. Der so entstandene dicke rote Brei wird auf 60°C erhitzt und dann allmählich im Verlauf von 110 Minuten mit 858 ml (6,6 Mol) α-Methylstyrol versetzt, währenddessen die Temperatur von 60 auf 90°C steigt. Das tiefrote flüssige Reaktionsgemisch wird noch weitere 200 Minuten auf 90—105°C erhitzt, bis nur eine Spur o-Nitroanilin durch Dünnschicht-chromatographie nachweisbar ist.

Das heisse Reaktionsgemisch verdünnt man mit 3 Liter Toluol und rührt, trennt die wässrige Phase bei 60°C ab und wäscht die Toluolphase 4-mal mit je 700 ml Wasser. Die Toluolphase wird filtriert und das Filtrat über wasserfreiem Natriumsulfat getrocknet. Die trockene Toluollösung erhitzt man auf 70°C, versetzt mit 4 Liter Heptan in 1-Liter Anteilen, um die Temperatur über 55°C zu halten, und kühlt dann ab, was 4-α-Cumyl-2-nitroanilin als rotorangefarbige Kristalle vom Schmelzpunkt 92—94°C in 976 g Ausbeute (69,2%) liefert.

## Beispiel 2

4-α-Methylbenzyl-2-nitroanilin

Verfährt man wie in Beispiel 1, jedoch unter Ersatz des α-Methylstyrols durch eine äquivalente Menge Styrol, so erhält man 4-α-Methylbenzyl-2-nitroanilin als Kristalle vom Schmelzpunkt 95,5—97°C.

## Beispiel 3

4-Benzyl-2-nitroanilin

Verfährt man wie in Beispiel 1, jedoch unter Ersatz des α-Methylstyrols durch eine äquivalente Menge Benzylalkohol, so erhält man 4-Benzyl-2-nitroanilin.

## Beispiel 4

2,4-Di-(α,α-dimethylbenzyl)-phenol

Dieses Zwischenprodukt wird durch Umsetzung eines Gemischs aus 705,8 g (7,5 Mol) Phenol mit 1772,7 g (15 Mol) α-Methylstyrol in Gegenwart von 25,7 g (0,135 Mol) p-Toluolsulfonsäuremonohydrat als Katalysator hergestellt. Man erhitzt dieses Gemisch unter Stickstoff 2,5 Stunden lang auf 140°C, kühlt auf 110°C und versetzt mit 1125 ml Toluol. Nach Waschen der entstandenen Lösung bei 80°C mit 750 ml einer

wässrigen Lösung von 37,5 g Natriumcarbonat und 75 g Natriumchlorid wäscht man die organische Phase dreimal mit je 1000 ml wässriger Natriumchloridlösung, trocknet über wasserfreiem Natriumsulfat, filtriert und destilliert im Vakuum. Dabei erhält man das obengenannte Produkt als die bei 172—175°C/ 0,15—0,18 mm Hg siedende Hauptfraktion in 1229,8 g Ausbeute (49,6% der Theorie). Das Produkt schmilzt bei 63—65°C.

Beispiel 5

4-α,α-Dimethylbenzyl-2-nitrobenzoldiazoniumchlorid

In einem mit Rührer und Thermometer ausgerüsteten 500 ml-Kolben suspendiert man 76,9 g (0,3 Mol) 4-, -Dimethylbenzyl-2-nitroanilin in 200 ml Xylol und versetzt bei 25°C mit 86,4 g (0,9 Mol) konzentrierter Salzsäure. Die Suspension wird 1 Stunde lang bei 38°C gerührt und dann mit 60 ml Wasser versetzt. Man kühlt auf 20°C und impft, worauf das entsprechende Hydrochloridsalz als körniger Niederschlag kristallisiert. Man kühlt auf −5°C und diazotiert durch Zusatz von 21,4 g (0,31 Mol) festem Natriumnitrit im Verlauf von 30 Minuten, wobei man die Temperatur auf −5°C bis −2°C hält, und rührt noch 1 Stunde bei −2°C. Es bilden sich zwei Phasen, und diese werden getrennt. Die untere wässrige Phase enthält das gewünschte Diazoniumchlorid und wiegt 244,1 g, entsprechend 0,3 Mol Diazoniumchloridlösung.

Beispiel 6

4-α,α-Dimethylbenzyl-2-nitro-2′-hydroxy-3′,5′-di-tert.-butylazobenzol

In einem 1 Liter-Kolben löst man 54 g (1,35 Mol) Natriumhydroxidplätzchen in 500 ml Methanol und versetzt mit 51,6 g (0,25 Mol) 2,4-Di-tert.butylphenol und 50 ml Xylol. Die so erhaltene Lösung wird auf −8°C gekühlt und im Verlauf von 6 Stunden mit 244 g (0,3 Mol) der in Beispiel 5 hergestellten Diazoniumchloridlösung versetzt, wobei man die Temperatur bei −8°C hält. Das Reaktionsgemisch wird über Nachtgerührt, und der pH durch Zusatz von 55 ml (0,68 Mol) konzentrierter Salzsäure auf 9 gestellt.

Die überstehende Lösung wird von dem halbfesten Rückstand entfernt, welcher das rohe Azobenzolprodukt darstellt, das zur Entfernung anorganischer Salze dreimal mit je 100 ml Methanol und dreimal mit je 100 ml Wasser aufgeschlämmt wird. Den erhaltenen festen Rückstand behandelt man mit 300 ml Methanol, was diesen zur Kristallisation bringt. Nach Filtrieren bei 15°C erhält man das Azobenzolprodukt in 82,8 g Ausbeute (69,9%) als ziegelroten Feststoff. Nach Umkristallisieren aus Ethanol schmilzt das Produkt bei 105—107°C.

Beispiel 7

5-α,α-Dimethylbenzyl-2-(2-hydroxy-3,5-di-tert.butylphenyl)-2H-benztriazol

Einen mit Rührer, Thermometer, Rückflusskühler und Stickstoffeinlass ausgerüsteten 1 Liter-Dreihalskolben beschickt man mit 101,5 g (0,214 Mol) o-Nitroazobenzolzwischenprodukt aus Beispiel 6 und 430 ml Toluol. Die entstandene Lösung versetzt man mit 64,2 ml Isopropanol und 64,2 ml Wasser, setzt das Gemisch unter Stickstoff und gibt 34,2 g (0,43 Mol) 50%ige wässrige Natronlauge dazu. Ein 49,2 g (0,75 Grammatom) Zink enthaltender Kolben wird mit dem Reaktionskolben mittels eines Gooch-Gummischlauchs verbunden, und der Zinkstaub wird portionsweise dem Reaktionsgemisch in 7 gleichen Anteilen mit 30 Minuten Zwischenräumen zwischen den Zugaben zugesetzt. Wenn alles Zink zugegeben ist, rührt man über Nacht bei 50°C und erhitzt dann 2 Stunden lang auf 75—80°C. Dann kühlt man auf 45°C ab und säuert mit 214 g 50%iger wässriger Schwefelsäure an.

Der Zinkschlamm wird durch Filtrieren entfernt. Das Produkt befindet sich in der organischen Schicht, die fünfmal mit je 30 ml 70%iger Schwefelsäure gewaschen und dann über wasserfreiem Magnesiumsulfat getrocknet wird. Die Toluollösung engt man auf 150 g ein, verdünnt dann mit 200 ml Ethanol und impft, was das obengenannte Produkt als weissen Feststoff vom Schmelzpunkt 93—95°C in 52,5 g Ausbeute (55,6%) ergibt.

Analyse:

| | | | |
|---|---|---|---|
| Berechnet für $C_{29}H_{35}N_3O$: | C, 78,87; | H, 7,99; | N, 9,52. |
| Gefunden: | C, 78,5; | H, 8,2; | N, 9,4. |

Beispiele 8—9

Verfährt man wie in Beispiel 5, unter Ersatz des 4-α,α-Dimethylbenzyl-2-nitroanilins durch eine äquivalente Menge 4-α-Methylbenzyl-2-nitroanilin (Beispiel 8) bzw. 4-Benzyl-2-nitroanilin (Beispiel 9), so erhält man die entsprechende Diazoniumchloridlösung.

Beispiel 10—20

o-Nitroazobenzol-Produkte

Zur Herstellung der folgenden o-Nitroazobenzolzwischenprodukte verfährt man wie in Beispiel 6 unter Verwendung der entsprechenden Diazoniumchloridlösung und des entsprechenden Phenols, das beispielsweise analog Beispiel 4 hergestellt wird.

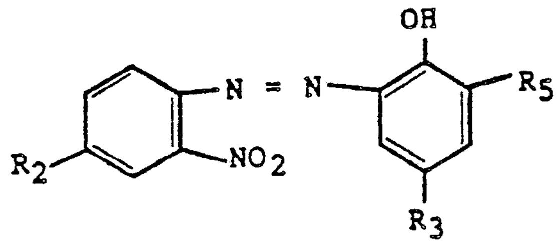

| Beispiel | $R_2$ | $R_3$ | $R_5$ |
|---|---|---|---|
| 10 | α-Cumyl* | α-Cumyl | α-Cumyl |
| 11 | α-Cumyl* | tert.Octyl | α-Cumyl |
| 12 | α-Cumyl* | tert.Amyl | tert.Amyl |
| 13 | α-Cumyl* | tert.Octyl | tert.Octyl |
| 14 | α-Methylbenzyl** | α-Cumyl | α-Cumyl |
| 15 | Benzyl*** | α-Cumyl | α-Cumyl |
| 16 | α-Cumyl* | Methyl | Wasserstoff |
| 17 | α-Cumyl* | tert.Octyl | Wasserstoff |
| 18 | α-Cumyl* | α-Cumyl | tert.Octyl |
| 19 | α-Cumyl* | Methyl | tert.Butyl |
| 20 | α-Cumyl* | tert.Butyl | Wasserstoff |

* aus Diazoniumchlorid nach Beispiel 5

** aus Diazoniumchlorid nach Beispiel 8

*** aus Diazoniumchlorid nach Beispiel 9

Beispiel 21

5-α,α-Dimethylbenzyl-2-[2-hydroxy-3,5-di-(α,α-dimethylbenzyl)-phenyl]-2H-benztriazol

Verfährt man wie in Beispiel 7 unter Verwendung des o-Nitroazobenzolzwischenprodukts aus Beispiel 10, so erhält man das obengenannte Produkt in 59,9% Ausbeute als weissen Feststoff vom Schmelzpunkt 141—142°C.

Analyse:

| | | | |
|---|---|---|---|
| Berechnet für $C_{39}H_{39}N_3O$: | C, 82,79; | H, 6,95; | N, 7,43. |
| Gefunden: | C, 82,9; | H, 7,2; | N, 7,5. |

Das Produkt aus Beispiel 21 erhält man in zwei verschiedenen Kristallmodifikationen, von denen eine bei 114—116°C schmilzt und eine weit höher Löslichkeit in zahlreichen organischen Lösungsmitteln, einschliesslich Xylol und Methylamylketon, aufweist als die andere Kristallform.

Beispiel 22

5-α,α-Dimethylbenzyl-2-(2-hydroxy-3-α,α-dimethylbenzyl-5-tert.octylphenyl)-2H-benztriazol

Verfährt man wie in Beispiel 7 unter Verwendung des o-Nitroazobenzolzwischenprodukts aus Beispiel 11, so erhält man das obengenannte Produkt in 34% Ausbeute als bernsteinfarbenes Glas, das beim Umkristallisieren einen Feststoff vom Schmelzpunkt 99—100°C ergibt.

Analyse:

| | | | |
|---|---|---|---|
| Berechnet für $C_{38}H_{45}N_3O$: | C, 81.53; | H, 8,10; | N, 7,51. |
| Gefunden: | C, 81,3; | H, 8,3; | N, 7,5. |

Beispiel 23

5-α,α-Dimethylbenzyl-2-(2-hydroxy-3,5-Di-tert.amylphenyl)-2H-benztriazol

Verfährt man wie in Beispiel 7 unter Verwendung des o-Nitroazobenzolzwischenprodukts aus Beispiel

12, so erhält man das obengenannte Produkt in 34% Ausbeute als bernsteinfarbenes Glas.

Analyse:

| | | | |
|---|---|---|---|
| Berechnet für $C_{31}H_{39}N_3O$: | C, 79,27; | H, 8,37; | N, 8,95. |
| Gefunden: | C, 80,2; | H, 8,5; | N, 8,9. |

Beispiel 24

5-α,α-Dimethylbenzyl-2-(2-hydroxy-3,5-di-tert.octylphenyl)-2H-benztriazol

Verfährt man wie in Beispiel 7 unter Verwendung des o-Nitroazobenzolzwischenprodukts aus Beispiel 13, so erhält man das obengenannte Produkt als Feststoff, der nach Umkristallisieren aus Isopropanol bei 120—122°C schmilzt.

Analyse:

| | | | |
|---|---|---|---|
| Berechnet für $C_{37}H_{51}N_3O$: | C, 80,24; | H, 9,28; | N, 7,59. |
| Gefunden: | C, 80,3; | H, 9,3; | N, 7,7. |

Beispiel 25

5-α-Methylbenzyl-2-[2-hydroxy-3,5-di-(α-α-dimethylbenzyl)-phenyl]-2H-benztriazol

Verfährt man wie in Beispiel 7 unter Verwendung des o-Nitroazobenzolzwischenprodukts aus Beispiel 14, so erhält man die obengenannte Verbindung in 44,4% Ausbeute als Feststoff vom Schmelzpunkt 123—125°C.

Analyse:

| | | | |
|---|---|---|---|
| Berechnet für $C_{38}H_{37}N_3O$: | C, 82,72; | H, 6,76; | N, 7,62. |
| Gefunden: | C, 82,6; | H, 6,7; | N, 7,6. |

Beispiel 26

5-Benzyl-2-[2-hydroxy-3,5-di-(α,α-dimethylbenzyl)-phenyl]-2H-benztriazol

Verfährt man wie in Beispiel 7 unter Verwendung des o-Nitroazobenzolzwischenprodukts aus Beispiel 15, so erhält man das obengenannte Produkt in 44,6% Ausbeute als weissen Feststoff vom Schmelzpunkt 91—93°C.

Analyse:

| | | | |
|---|---|---|---|
| Berechnet für $C_{37}H_{35}N_3O$: | C, 82,65; | H, 6,56; | N, 7,82. |
| Gefunden: | C, 82,5; | H, 6,4; | N, 7,8. |

Beispiel 27

5-α,α-Dimethylbenzyl-2-(2-hydroxy-3-tert.butyl-5-methylphenyl)-2H-benztriazol

Verfährt man wie in Beispiel 7 unter Verwendung des o-Nitroazobenzolzwischenprodukts aus Beispiel 19, so erhält man die obengenannte Verbindung als Feststoff vom Schmelzpunkt 87—89°C.

Analyse:

| | | | |
|---|---|---|---|
| Berechnet für $C_{26}H_{29}N_3O$: | C, 78,16; | H, 7,32; | N, 10,52. |
| Gefunden: | C, 77,9; | H, 7,5; | N, 10,3. |

Beispiele 28—31

Verfährt man wie in Beispiel 7 unter Verwendung der entsprechenden o-Nitroazobenzolzwischenprodukte aus den Beispielen 16—18 bzw. 20, so erhält man die folgenden 2H-Benztriazole.

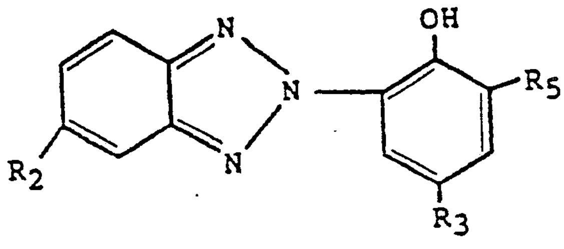

| Beispiel | $R_2$ | $R_3$ | $R_5$ |
|---|---|---|---|
| 28 | α-Cumyl | Methyl | Wasserstoff |
| 29 | α-Cumyl | tert.Octyl | Wasserstoff |
| 30 | α-Cumyl | α-Cumyl | tert.Octyl |
| 31 | α-Cumyl | tert.Butyl | Wasserstoff |

Beispiel 32

Widerstandsfähigkeit gegen Verlust an Benztriazolstabilisatoren

Die 2-(2-Hydroxyphenyl)-2H-benztriazollichtschutzmittel aus Beispielen 7, 21—24 und 26 werden der thermogravimetrischen Analyse bei einer Durchflussgeschwindigkeit von 100 ml Stickstoff/Minute sowohl isotherm bei 280°C, um die Zeit in Minuten anzugeben, bei der 50% Gewichtsverlust an Stabilisator erreicht wird, als auch mit Differentialabtastung bei einer Heizgeschwindigkeit von 10°C pro Minute, um die Temperatur zu bestimmen, bei der man 10% bzw. 50% Gewichtsverlust an Stabilisator beobachtet, unterzogen.

Die Versuchsdaten sind in der nachfolgenden Tabelle angegeben.

Diese Ergebnisse stehen in naher Beziehung zu der Widerstandsfähigkeit des jeweiligen Stabilisators gegen Ausschwitzen oder Verlüchtigung während irgendeinem Verarbeitungsvorgang mit Polymerformulierungen bei der Herstellung von Folien, Fasern oder sonstigen gefertigten Flächengebilden. Die Abwesenheit oder weitgehende Abwesenheit ausgeschwitzten oder verflüchtigten Stabilisators auf den Verarbeitungsgeräten (d.h. Walzen, Führungen, Öffnungen und dergleichen) verlängert erheblich die Zeiten zwischen erforderlichen Abstellungen in kontinuierlich betriebenen Verarbeitungsgeräten und führt zu enormen praktischen und wirtschaftlichen Ersparnissen bezüglich des jeweils verwendeten Stabilisators.

| | Thermogravimetrische Analysendaten | | |
|---|---|---|---|
| Stabilisator aus Beispiel | Isotherm bei 280°C Zeit (Minuten) bis zum angegebenen Gewichtsverlust an Stabilisator | Abtasten (bei 10°C pro Minute) Temperatur in °C bis zum angegebenen Gewichtsverlust an Stabilisator | |
| | 50 % | 10 % | 50 % |
| 7 | 19 | 295 | 338 |
| 21 | 2040 | 365 | 412 |
| 22 | 840 | 340 | 387 |
| 23 | 33 | 285 | 325 |
| 24 | 40 | 308 | 348 |
| 26 | 3780 | 360 | 405 |

Die Benztriazole aus Beispielen 7, 21—24 und 26 sind sehr widerstandsfähig gegen thermogravimetrischen Verlust. Dies bedeutet, dass sie ausgezeichnete Widerstandsfähigkeit gegen Sublimation und Ausschwitzen aufweisen.

Die vorliegenden Verbindungen würden bei Einarbeitung in eine stabilisierte Polymerzusammensetzung bei der Verarbeitung in dieser verbleiben und somit zu ausgezeichneter Verarbeitungsfähigkeit sowie auch einem fertigen polymeren Flächengebilde mit besserem Schutz gegen späteren lichtinduzierten Abbau führen.

Beispiel 33

Löslichkeit der Benztriazolstabilisatoren

Benztriazolstabilisatoren sind herkömmlicherweise Stoffe mit verhältnismässig geringer Löslichkeit in üblichen organischen Lösungsmitteln. Dies gilt besonders, wenn das Benztriazol im 2-Phenylring mit Aralkylgruppen substituiert ist, welche die Flüchtigkeit des Stabilisators bei der Verarbeitung und im Gebrauch verringern sollen.

Es wurde nun überraschend gefunden, dass die Verbindungen der Formel I gute Löslichkeit in verschiedenen Lösungsmitteln aufweisen.

Die Löslichkeitsdaten sind unten in der Tabelle angegeben.

| Stabilisator aus Beispiel | Löslichkeit von Benztriazolstabilisatoren in g pro 100 g Lösungsmittel bei 25°C | | | |
|---|---|---|---|---|
| | Heptan | Xylol | Methyl-amyl-keton | Butyl-cellosolve-acetat |
| 7 | 97 | 197 | 128 | 61 |
| 23 | >100 | >100 | >100 | >100 |

Beispiel 34

Absorption bei 340 nm

Die erfindungsgemässen Verbindungen zeigen sehr hohe Absorption bei 340 nm, wie aus der Bestimmung des molaren Extinktionskoeffizienten E ersichtlich ist.

| Verbindung aus | Molarer Extinktions-koeffizient E |
|---|---|
| Beispiel 7 | 19 000 |
| Beispiel 21 | 19 500 |
| Beispiel 22 | 18 500 |
| Beispiel 23 | 19 100 |
| Beispiel 24 | 17 400 |
| Beispiel 26 | 18 500 |

Beispiel 35

Ein heisshärtbarer Acryleinbrennlack mit hohem Feststoffgehalt, bestehend aus 70 Gewichtsteilen eines aus Methylmethacrylat, Hydroxyethylmethacrylat, Butylacrylat und Styrol hergestellten Copolymeren, 30 Gewichtsteilen Hexakismethoxymethylmelamin als Vernetzungsmittel und 0,1 Gewichtsteilen p-Toluolsulfonsäure, wird mit 2 Gewichtsteilen der Verbindungen aus Beispiel 22—24 formuliert. Der so erhaltene klare Einbrennlack wird dann als Klarlack auf mit Silbermetalleffektlack grundierte Stahltafeln gespritzt. Für Vergleichszwecke wird eine Probe ohne Stabilisator hergestellt.

Nach der Härtung werden die Tafeln dann im Freien in Florida für einen Zeitraum von 12 Monaten ausgesetzt. Danach wird der noch verbleibende Glanz (in %) bestimmt:

| Stabilisator | % des ursprünglichen Glanzes |
|---|---|
| Ohne Stabilisator | 30 |
| Verbindung aus | |
| Beispiel 22 | 73 |
| Beispiel 23 | 76 |
| Beispiel 24 | 82 |

Beispiel 36

Proben der Lichtschutzmittel werden durch 15 Minuten langes Walzen bei 24°C in Polycarbonatharz eingearbeitet. Während dieser Walzzeit geht etwas Lichtschutzmittel je nach dessen Flüchtigkeit und

Verträglichkeit durch Sublimation verloren. Der prozentuale Stabilisatorverlust nach der Walzzeit wird spektrophotometrisch bestimmt.

| | % Gewichtsverlust |
|---|---|
| Stabilisator aus Beispiel 7 | <3 |
| Stabilisator aus Beispiel 21 | <3 |

## Patentansprüche

1. Verbindungen der Formel I

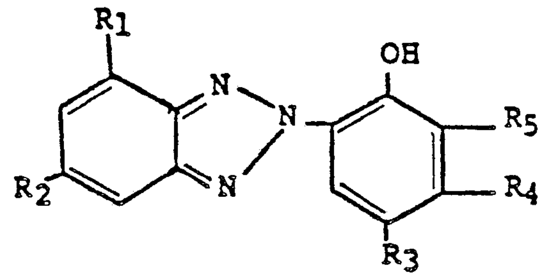

(I)

worin $R_1$ und $R_2$ unabhängig voneinander Wasserstoff, Halogen, Alkyl mit 1 bis 18 Kohlenstoffatomen oder eine Gruppe der Formel II

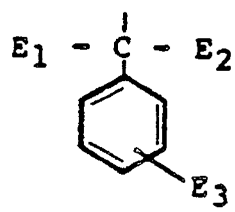

(II)

wobei $E_1$ und $E_2$ unabhängig voneinander für Wasserstoff oder Alkyl mit 1 bis 4 Kohlenstoffatomen und $E_3$ für Wasserstoff, Halogen oder Alkyl mit 1 bis 4 Kohlenstoffatomen stehen, mit der Massgabe, dass mindestens eines von $R_1$ und $R_2$ eine Gruppe der Formel II sein muss; $R_3$ Wasserstoff, Hydroxyl, Alkyl mit 1 bis 12 Kohlenstoffatomen, Alkoxy mit 1 bis 4 Kohlenstoffatomen, gegebenenfalls durch eine, zwei oder drei Alkylgruppen mit 1 bis 8 Kohlenstoffatomen substituiertes Phenyl, Cycloalkyl mit 5 bis 6 Kohlenstoffatomen, Carboalkoxy mit 2 bis 9 Kohlenstoffatomen, Chlor, Carboxyethyl oder eine Gruppe der Formel II, $R_4$ Wasserstoff, Alkyl mit 1 bis 4 Kohlenstoffatomen, Alkoxy mit 1 bis 12 Kohlenstoffatomen, Chlor oder Hydroxyl und $R_5$ Wasserstoff, Hydroxyl, Alkyl mit 1 bis 12 Kohlenstoffatomen, Chlor, Cycloalkyl mit 5 bis 6 Kohlenstoffatomen oder eine Gruppe der Formel II bedeuten, mit der Massgabe, dass $R_3$, $R_4$ und $R_5$ nicht gleichzeitig alle für Wasserstoff stehen dürfen, und dass jeweils nur eines von $R_3$, $R_4$ und $R_5$ Hydroxyl darstellen kann.

2. Verbindungen nach Anspruch 1, dadurch gekennzeichnet, dass $R_1$ und $R_2$ unabhängig voneinander Wasserstoff, Alkyl mit 1 bis 12 Kohlenstoffatomen oder eine Gruppe der Formel II, wobei $E_1$ und $E_2$ unabhängig voneinander für Wasserstoff, Methyl oder Ethyl und $E_3$ für Wasserstoff, Chlor, Methyl oder Ethyl stehen, $R_3$ Wasserstoff, Alkyl mit 1 bis 12 Kohlenstoffatomen oder eine Gruppe der Formel II, $R_4$ Wasserstoff und $R_5$ Wasserstoff, Alkyl mit 1 bis 12 Kohlenstoffatomen oder eine Gruppe der Formel II bedeuten.

3. Verbindungen nach Anspruch 1, dadurch gekennzeichnet, dass nur eines von $R_1$ und $R_2$ eine Gruppe der Formel II bedeutet.

4. Verbindungen nach Anspruch 1, dadurch gekennzeichnet, dass $R_1$ Wasserstoff und $R_2$ eine Gruppe der Formel II bedeutet.

5. Verbindungen nach Anspruch 1, dadurch gekennzeichnet, dass $R_1$ Wasserstoff und $R_2$ eine Gruppe der Formel II, worin mindestens eines von $E_1$ und $E_2$ für Methyl und $E_3$ für Wasserstoff oder p-Methyl stehen, bedeuten.

6. Verbindungen nach Anspruch 5, dadurch gekennzeichnet, dass $R_2$ α,α-Dimethylbenzyl bedeutet.

7. Verbindungen nach Anspruch 1, dadurch gekennzeichnet, dass $R_3$ Alkyl mit 1 bis 8 Kohlenstoffatomen oder eine Gruppe der Formel II bedeutet.

8. Verbindungen nach Anspruch 7, dadurch gekennzeichnet, dass $R_3$ Methyl, tert.Butyl, tert.Amyl, tert.Octyl, sek.Butyl, Benzyl, α-Methylbenzyl oder α,α-Dimethylbenzyl bedeutet.

9. Verbindungen nach Anspruch 1, dadurch gekennzeichnet, dass $R_4$ Wasserstoff, Hydroxyl, Alkyl mit 1 bis 4 Kohlenstoffatomen oder Alkoxy mit 1 bis 8 Kohlenstoffatomen bedeutet.

10. Verbindungen nach Anspruch 9, dadurch gekennzeichnet, dass $R_4$ Wasserstoff bedeutet.

11. Verbindungen nach Anspruch 1, dadurch gekennzeichnet, dass $R_5$ Wasserstoff, Alkyl mit 1 bis 8 Kohlenstoffatomen oder eine Gruppe der Formel II bedeutet.

12. Verbindungen nach Anspruch 11, dadurch gekennzeichnet, dass $R_5$ Wasserstoff, Methyl, sek.Butyl, tert.Butyl, tert.Amyl, tert.Octyl, Benzyl, α-Methylbenzyl oder α,α-Dimethylbenzyl bedeutet.

13. Zusammensetzungen, enthaltend ein organisches, licht-induziertem Abbau unterliegendes Substrat und mindestens eine Verbindung der Formel I nach Anspruch 1.

14. Zusammensetzungen nach Anspruch 13, worin das organische Substrat ein synthetisches Polymer ist.

15. Zusammensetzungen nach Anspruch 14, worin das Polymer ein Aminoplast ist.

16. Zusammensetzungen nach Anspruch 15, worin das Aminoplast ein heisshärtbares Acrylharz ist.

17. Verbindungen der Formel

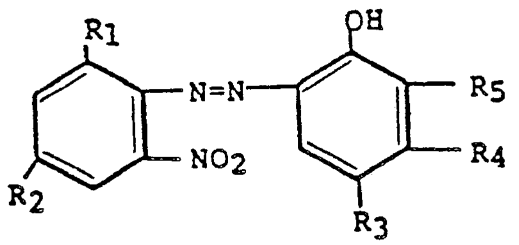

(III)

worin $R_1$, $R_2$, $R_3$, $R_4$ und $R_5$ wie in Anspruch 1 definiert sind.

**Revendications**

1. Composés répondant à la formule I:

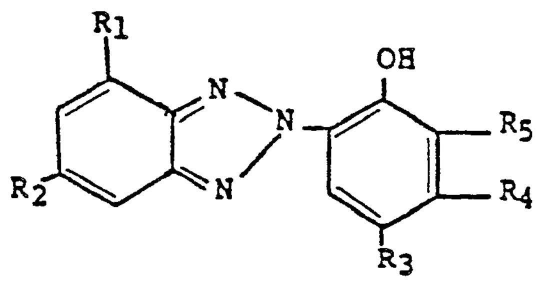

(I)

dans laquelle:

$R_1$ et $R_2$ représentent chacun, indépendamment l'un de l'autre, l'hydrogène, un halogène, un alkyle contenant de 1 à 18 atomes de carbone ou un radical répondant à la formule II:

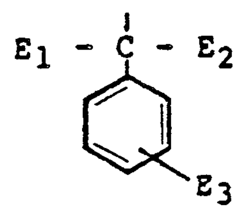

(II)

dans laquelle $E_1$ et $E_2$ représentent chacun, indépendamment l'un de l'autre, l'hydrogène ou un alkyle contenant de 1 à 4 atomes de carbone, et $E_3$ représente l'hydrogène, un halogène ou un alkyle contenant de 1 à 4 atomes de carbone, avec la condition qu'au moins l'un des symboles $R_1$ et $R_2$ représente un radical de formule II,

$R_3$ représente l'hydrogène, un hydroxy, un alkyle contenant de 1 à 12 atomes de carbone, un alcoxy contenant de 1 à 4 atomes de carbone, un phényle éventuellement porteur d'un, de deux ou de trois alkyles en $C_1$—$C_8$, un cycloalkyle contenant 5 ou 6 atomes de carbone, un alcoxycarbonyle contenant de 2 à 9 atomes de carbone, le chlore, un carboxyéthyle ou un radical de formule II,

$R_4$ représente l'hydrogène, un alkyle contenant de 1 à 4 atomes de carbone, un alcoxy contenant de 1 à 12 atomes de carbone, le chlore ou un hydroxy, et

$R_5$ représente l'hydrogène, un hydroxy, un alkyle contenant de 1 à 12 atomes de carbone, le chlore, un

cycloalkyle contenant 5 ou 6 atomes de carbone ou un radical de formule II, avec la condition que $R_3$, $R_4$ et $R_5$ ne représentent pas tous les trois en même temps un atome d'hydrogène et que l'un seulement des symboles $R_3$, $R_4$ et $R_5$ puisse représenter un hydroxy.

2. Composés selon la revendication 1 caractérisés en ce que:

$R_1$ et $R_2$ représentent chacun, indépendamment l'un de l'autre, l'hydrogène, un alkyle contenant de 1 à 12 atomes de carbone ou un radical de formule II dans lequel $E_1$ et $E_2$ représentent chacun, indépendamment l'un de l'autre, l'hydrogène, un méthyle ou un éthyle et $R_3$ représente l'hydrogène, le chlore, un méthyle ou un éthyle,

$R_3$ représente l'hydrogène, un alkyle contenant de 1 à 12 atomes de carbone ou un radical de formule II,

$R_4$ représente l'hydrogène et

$R_5$ représente l'hydrogène, un alkyle contenant de 1 à 12 atomes de carbone ou un radical de formule II.

3. Composés selon la revendication 1 caracterisés en ce que l'un seulement des symboles $R_1$ et $R_2$ représente un radical de formule II.

4. Composés selon la revendication 1 caracterisés en ce que $R_1$ représente l'hydrogène et $R_2$ un radical de formule II.

5. Composés selon la revendication 1 caracterisés en ce que $R_1$ représente l'hydrogène et $R_2$ un radical de formule II dans lequel au moins l'un des symboles $E_1$ et $E_2$ représente un méthyle et $E_3$ représente l'hydrogène ou un radical méthyle en position para.

6. Composés selon la revendication 5 caractérisés en ce que $R_2$ représente un radical α,α-diméthylbenzyle.

7. Composés selon la revendication 1 caractérisés en ce que $R_3$ représente un alkyle contenant de 1 à 8 atomes de carbone ou un radical de formule II.

8. Composés selon la revendication 7 caractérisés en ce que $R_3$ représente un radical méthyle, tert-butyle, tert-pentyle, tert-octyle, sec-butyle, benzyle, α-méthylbenzyle ou α,α-diméthylbenzyle.

9. Composés selon la revendication 1 caractérisés en ce que $R_4$ représente l'hydrogène, un hydroxy, un alkyle contenant de 1 à 4 atomes de carbone ou un alcoxy contenant de 1 à 8 atomes de carbone.

10. Composés selon la revendication 9 caractérisés en ce que $R_4$ représente l'hydrogène.

11. Composés selon la revendication 1 caractérisés en ce que $R_5$ représente l'hydrogène, un alkyle contenant de 1 à 8 atomes de carbone ou un radical de formule II.

12. Composés selon la revendication 11 caractérisés en ce que $R_5$ représente l'hydrogène ou un radical méthyle, sec-butyle, tert-butyle, tert-pentyle, tert-octyle, benzyle, α-méthylbenzyle ou α,α-diméthylbenzyle.

13. Compositions contenant un substrat organique risquant d'être dégradé sous l'effet de la lumière et au moins un composé de formule I selon la revendication 1.

14. Compositions selon la revendication 13 dans lesquelles le substrat organique est un polymère synthétique.

15. Compositions selon la revendication 14 dans lesquelles le polymère est un aminoplaste.

16. Compositions selon la revendication 15 dans lesquelles l'aminoplaste est un résine acrylique thermodurcissable.

17. Composés répondant à la formule:

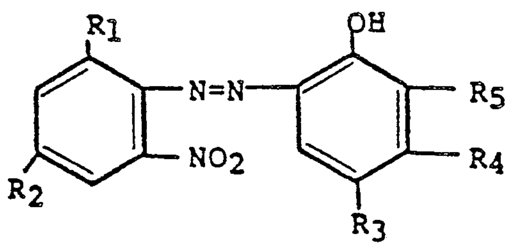

dans laquelle $R_1$, $R_2$, $R_3$, $R_4$ et $R_5$ ont les significations qui leur ont été données à la revendication 1.

**Claims**

1. A compound of formula I

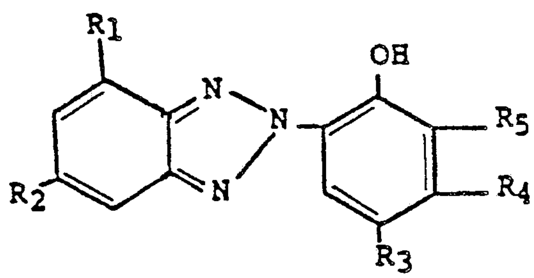

(I)

in which $R_1$ and $R_2$ are independently of one another hydrogen, halogen, alkyl of 1 to 18 carbon atoms or a group of formula II

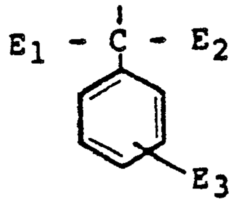

(II)

where $E_1$ and $E_2$ are independently of one another hydrogen or alkyl of 1 to 4 carbon atoms and $E_3$ is hydrogen, halogen or alkyl of 1 to 4 carbon atoms, with the proviso that at least one of $R_1$ and $R_2$ must be a group of formula II; $R_3$ is hydrogen, hydroxyl, alkyl of 1 to 12 carbon atoms, alkoxy of 1 to 4 carbon atoms, phenyl which is unsubstituted or substituted by one, two or three alkyl groups having 1 to 8 carbon atoms, cycloalkyl of 5 to 6 carbon atoms, carboalkoxy of 2 to 9 carbon atoms, chlorine, carboxyethyl or a group of formula II, $R_4$ is hydrogen, alkyl of 1 to 4 carbon atoms, alkoxy of 1 to 12 carbon atoms, chlorine or hydroxyl, and $R_5$ is hydrogen, hydroxyl, alkyl of 1 to 12 carbon atoms, chlorine, cycloalkyl of 5 to 6 carbon atoms or a group of formula II, with the proviso that $R_3$, $R_4$ and $R_5$ cannot all be hydrogen at the same time, and that only one of $R_3$ $R_4$ and $R_5$ can be hydroxyl at the same time.

2. A compound according to claim 1 wherein $R_1$ and $R_2$ are independently of one another hydrogen, alkyl of 1 to 12 carbon atoms or a group of formula II where $E_1$ and $E_2$ are independently of one another hydrogen, methyl or ethyl and $E_3$ is hydrogen, chlorine, methyl or ethyl, $R_3$ is hydrogen, alkyl of 1 to 12 carbon atoms or a group of formula II, $R_4$ is hydrogen and $R_5$ is hydrogen, alkyl of 1 to 12 carbon atoms or a group of formula II.

3. A compound according to claim 1 wherein only one of $R_1$ and $R_2$ is a group of formula II.

4. A compound according to claim 1 wherein $R_1$ is hydrogen and $R_2$ is a group of formula II.

5. A compound according to claim 1 wherein $R_1$ is hydrogen, $R_2$ is a group of formula II in which at least one of $E_1$ and $E_2$ is methyl and $E_3$ is hydrogen or p-methyl.

6. A compound according to claim 5 wherein $R_2$ is α,α-dimethylbenzyl.

7. A compound according to claim 1 wherein $R_3$ is alkyl of 1 to 8 carbon atoms or a group of formula II.

8. A compound according to claim 7 wherein $R_3$ is methyl, tert-butyl, tert-amyl, tert-octyl, sec-butyl, benzyl, α-methylbenzyl or α,α-dimethylbenzyl.

9. A compound according to claim 1 wherein $R_4$ is hydrogen, hydroxyl, alkyl of 1 to 4 carbon atoms or alkoxy of 1 to 8 carbon atoms.

10. A compound according to claim 9 wherein $R_4$ is hydrogen.

11. A compound according to claim 1 wherein $R_5$ is hydrogen, alkyl of 1 to 8 carbon atoms or a group of formula II.

12. A compound according to claim 11 wherein $R_5$ is hydrogen, methyl, sec-butyl, tert-butyl, tert-amyl, tert-octyl, benzyl, α-methylbenzyl or α,α-dimethylbenzyl.

13. A composition containing an organic substrate subject to light-induced deterioration and at least one compound of formula I according to claim 1.

14. A composition according to claim 13 in which the organic substrate is a synthetic polymer.

15. A composition according to claim 14 in which the polymer is an aminoplast.

16. A composition according to claim 15 in which the aminoplast is a thermoset acrylic resin.

17. A compound of the formula

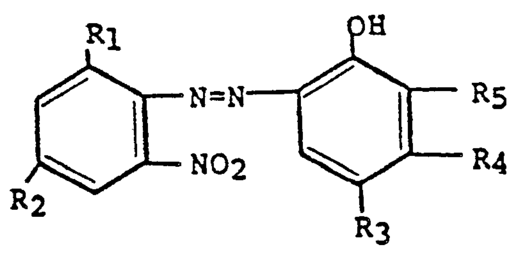

in which $R_1$, $R_2$, $R_3$, $R_4$ and $R_5$ are as defined in claim 1.